# EUROPEAN PATENT SPECIFICATION

(11) **EP 2 957 246 B1**
(45) Date of publication and mention of the grant of the patent: **19.04.2017**
(21) Application number: 14172835.2
(22) Date of filing: 17.06.2014
(51) Int. Cl.: A61B 17/70

(54) **Extension device for a bone anchor, in particular for minimally invasive surgery**
Verlängerungsvorrichtung eines Knochenankers, insbesondere für die minimalinvasive Chirurgie
Dispositif d'extension pour ancrage osseux, en particulier pour la chirurgie effractive minimale

(43) Date of publication of application: 23.12.2015
(73) Proprietor: Biedermann Technologies GmbH & Co. KG, 78166 Donaueschingen (DE)
(72) Inventor: Biedermann, Timo, 78647 Trossingen (DE); Dannecker, Berthold, 78112 St. Georgen (DE)
(74) Representative: Prüfer & Partner mbB Patentanwälte · Rechtsanwälte

(56) References cited:
- WO-A1-2013/187928
- US-A1- 2005 131 408
- US-A1- 2008 125 788
- US-A1- 2014 039 567
- US-A1- 2014 052 187
- US-B2- 7 563 264

## Description

The invention relates to an extension device for a bone anchor, in particular for use in minimally invasive surgery (MIS). The invention also relates to a system including such an extension device and a bone anchor, wherein the bone anchor comprises an anchoring section and a receiving part for receiving a rod to couple the rod to the anchoring section. The extension device includes a first sleeve and a second sleeve that are each configured to be coupled to the receiving part in such a manner that a translational and a rotational movement of the extension device relative to the receiving part is inhibited. A locking member is provided that guarantees correct decoupling of the sleeves from the receiving part.

Extension devices, also called head extenders, for pedicle screws for use in minimally invasive surgery are known in the art. For example, US 7,563,264 B2 describes a spinal stabilization system for a minimally invasive procedure wherein detachable sleeves may be coupled to a collar of a bone anchor to allow for formation of the spinal stabilization system through a small skin incision. The detachable sleeve members may allow for alignment of the collars to facilitate insertion of an elongated member in the collars. A coupling system is provided between the sleeve and the collar that inhibits translational movement of the sleeve relative to the collar. In one embodiment, the sleeve may be coupled to a collar of a bone fastener assembly with movable members that may be threaded into threaded openings in the collar.

WO 2013/112689 A2 describes a minimally invasive tower access device comprising an elongated outer sleeve that slidably receives an elongated inner sleeve. A lock nut is used to secure the inner sleeve and outer sleeve in a locked mode.

US 2005/0131408 A1 discloses percutaneous access devices and bone anchor anchor assemblies. The device has an outer tube, an inner tube and an adjustment mechanism allowing the operator to adjust the relative longitudinal position of the inner tube and the outer tube. The inner tube is inhibited from rotating with respect to the outer tube by means of a resilient tab provided midway on the inner tube, restricting mutual movement to that along the longitudinal direction in a limited range. Namely, the tab engages an elongate longitudinal slot provided in the outer tube. A receiving member of the bone anchor assembly is coupled by first rotating the whole device to bring the outer tube into coupling engagement with the receiving member, and then to displace the inner tube within the outer tube along the longitudinal direction with the resilient tab being in engagement with the slot, to couple also the inner tube to the receing member.

WO 2013/187928 A1 discloses a bone screw extension assembly. The assembly includes an inner and an outer sleeve. Relative rotation between the sleeves upon engagement with a bone screw assembly is prevented by a displaceable retention ring provided around a proximal end of the inner sleeve of the extension assembly. Thereby, the retention ring is held against rotation reative to the outer sleeve. Further, the retention ring is spring biased and can be axially displaced by forcibly driving an external socket driver against the retention ring with respect to a mating hex interface of the inner sleeve. Once the retention ring is sufficiently depressed in the proximal end of the inner tube, it gets clear of the hex interface and the inner sleeve may rotate. In this configuratuion, the inner tube may rotate with respect to the retention ring and thus with respect to the outer sleeve.

US 2008/0125788 A1 discloses a percutaneous instrument assembly. Each assembly has an outer shaft and an inner shaft which can be coupled to for allowing limited sliding relative to one another providing quick connection or disconnection for a fastener engagement member.

US 2014/0039567 A1 discloses a surgical apparatus. The apparatus comprises an inner sleeve and an outer sleeve. Commencing from a proximal end of the outer sleeve, opposed slots accommodate guidance projections projecting from the inner sleeve to inhibit twisting of the inner sleeve relative to the outer sleeve, when the projections engage in the slots.

US 2014/0052187 A1 discloses a spinal implant system. The system comprises an inner sleeve, an outer sleeve and an actuator configured for fixation with the outer sleeve and relatively movable engagement with inner sleeve to cause axial translation of the inner sleeve relative to the outer sleeve.

There is still a need for an extension device for a bone anchor that is not only safe during use in the surgical steps, such as compression and distraction, but that is also safe during the step of decoupling from the receiving part.

It is the object of the invention to provide an improved extension device for a bone anchor, in particular for use in minimally invasive surgery, and a system of such an extension device and a bone anchor that facilitates the surgical steps and improves the safety of the device.

The object is solved by a system of an extension device for a bone anchor according to claim 1 and by a spinal stabilization system according to claim 15. Further developments are given in the dependent claims.

The extension device is configured to be coupled to the receiving part such that it is locked against translational and rotational movement of the extension device relative to the receiving part. Because translational and rotational movements of the extension device and the receiving part are inhibited, the connection between the extension device and the receiving part is robust. This permits a safe placement of the rod and a set screw for fixing the rod. Surgical steps of adjustment of the spinal stabilization system, such as compression or distraction can be performed using the extension device attached to the receiving part, once the bone anchor has been inserted into the bone. In one embodiment, the coupling between the extension device and the receiving part is effected by a formfit engagement of a portion of the extension device with a portion of the receiving part. In another embodiment, the coupling is effected by a frictional engagement of a portion of the extension device with a portion of the receiving part. The coupling can also be partially a form-fit coupling and partially a friction-fit coupling.

The extension device comprises a first sleeve and a second sleeve positioned within the first sleeve and an interlocking bushing that connects the second sleeve to the first sleeve and allows a controlled axial movement of the second sleeve relative to the first sleeve. After the extension device has been attached to the receiving part, the first sleeve and the second sleeve can be interlocked with each other and with the receiving part by operating the interlocking bushing in one direction. The interlocking connection between the first sleeve and the second sleeve can be released by operating the interlocking bushing in the opposite direction.

In one embodiment, a translational movement between the extension device and the receiving part can be inhibited by a form-fit engagement of a circumferential rib that extends at least partially around the longitudinal axis of the device and engages a corresponding circumferential groove. The rib may be provided at the first sleeve and the groove at the receiving part or vice versa.

In another embodiment, the first sleeve may be coupled to the receiving part by means of a form-fit connection and the second sleeve may be coupled to the receiving part by a friction-fit conection only or vice versa

Decoupling of the extension device from the receiving part can be carried out only in a prescribed sequence of steps which in one embodiment consists in a first step of decoupling the second sleeve and a second step of decoupling the first sleeve. The correct sequence of steps is guaranteed by the presence of a locking member that, in a first configuration, inhibits the decoupling of the first sleeve when the second sleeve is still coupled to the receiving part. When the locking member is in a second configuration after decoupling the second sleeve, the decoupling of the first sleeve is allowed. Hence, a damage of the extension device based on erroneous operation is prevented. Moreover, with the use of such an extension device the surgeon can perform a next step in the surgical procedure only after correct decoupling of the extension device. This enhances the safety of the procedure.

The extension device may comprise a third sleeve that can be removably connected to the first sleeve in order to provide an extension device having an increased length. For example, a surgical procedure may be started with the longer extension device that comprises the third sleeve attached to the first sleeve for placement of the rod and the set screw. Thereafter, the third sleeve may be removed in order to improve the placement of the receiving part relative to the rod during compression and distraction steps. With the shorter extension device a greater variety of angles of the receiving parts relative to each other may be obtained.

The extension device comprises only few parts which facilitate the assembly and operation of the device.

The extension device may be used together with a release or decoupling instrument that is adapted to engage the extension device. The locking member of the extension device is configured to be engaged by the decoupling instrument in its second configuration only. It cannot be engaged in the first configuration in which both, the first and the second sleeve are coupled to the receiving part. Hence, the system of the extension device and the decoupling instrument guarantees the safety of handling and prevents damage of parts of the extension device or the receiving part.

As the spinal stabilization system includes at least two bone anchors with receiving parts that are adapted to be used with the extension device.

Further features and advantages of the invention will become apparent from the description of embodiments using the accompanying drawings.

In the drawings:
- Fig. 1: shows a perspective exploded view of the extension device according to a first embodiment.
- Fig. 2: shows a perspective partially exploded view of the extension device of Fig. 1 in an assembled state with an optional third sleeve.
- Fig. 3: shows a perspective view of the extension device of Fig. 2 in an assembled state.
- Fig. 4: shows a cross-sectional view of an upper portion of the extension device of Figs. 2 and 3 with the attached third sleeve, wherein the cross-section is taken in a plane including the sleeve axis.
- Fig. 5: shows a perspective view of an embodiment of a receiving part of a bone anchor that may form together with the extension device of Figs. 1 to 4 a first embodiment of a system of an extension device and a bone anchor.
- Fig. 6: shows a top view of the receiving part of Fig. 5.
- Fig. 7a: shows a cross-sectional view of the receiving part of Fig. 5 and Fig. 6 along the lines A-A' in Fig. 6.
- Fig. 7b: shows an enlarged view of a detail of Fig. 7a.
- Fig. 8a: shows a perspective view of a first sleeve of the extension device of Figs. 1 to 4.
- Fig. 8b: shows a perspective view from the bottom of a front end portion of the first sleeve of Fig. 8a.
- Fig. 9: shows a top view of the first sleeve of Fig. 8a.
- Fig. 10: shows a cross-sectional view of the first sleeve of Fig. 8a along line B-B in Fig. 9.
- Fig. 11: shows a cross-sectional view of the first sleeve of Fig. 8a along line D-D in Fig. 10.
- Fig. 12a: shows a perspective view of a second sleeve of the extension device of Figs. 1 to 4.
- Fig. 12b: shows an enlarged perspective view from the bottom of a front end portion of the second sleeve of Fig. 12a.
- Fig. 13: shows a cross-sectional view of the second sleeve according to Fig. 12a, the cross-section taken in a plane containing the sleeve axis and extending through the center of the legs of the sleeve.
- Fig. 14: shows an exploded perspective view of an interlocking bushing of the extension device of Figs. 1 and 2 and an end portion of the second sleeve.
- Fig. 15: shows a perspective view of the interlocking bushing and the end portion of the second sleeve of Fig. 14 in an assembled state.
- Fig. 16: shows a top view of the interlocking bushing of Fig. 14.
- Fig. 17: shows a cross-sectional view of the interlocking bushing of Fig. 14 along line E-E in Fig. 16.
- Fig. 18: shows a perspective view of a bone anchor with a receiving part and a front portion of the extension device according to the first embodiment coupled to the receiving part.
- Fig. 19: shows a cross-sectional view of an upper portion of the extension device, without third sleeve, and a front end portion of a decoupling instrument to be used with and adapted to the extension device, the cross-section taken in a plane including the sleeve axis.
- Fig. 20: shows a perspective exploded view of the third sleeve.
- Fig. 21: shows a top view of the third sleeve shown in Fig. 20.
- Fig. 22: shows a cross-sectional view of the third sleeve of Figs. 20 and 21 along line F-F in Fig. 21.
- Fig. 23a: shows a cross-sectional view of a first step of coupling the extension device to the receiving part of a bone anchor.
- Fig. 23b: shows a cross-sectional view of an upper portion of the extension device in the first step according to Fig. 23 a, wherein the interlocking bushing is in a second configuration.
- Fig. 24a: shows a cross-sectional view of a second step of coupling the extension device to the receiving part of a bone anchor.
- Fig. 24b: shows a cross-sectional view of an upper portion of the extension device in the step of Fig. 24a, wherein the interlocking bushing is still in the second configuration.
- Fig. 25a: shows a cross-sectional view of a third step of coupling the extension device to the receiving part of a bone anchor.
- Fig. 25b: shows a cross-sectional view of an upper portion of the extension device in the step of Fig. 25a, wherein the interlocking bushing is in a first configuration.
- Fig. 26: shows a perspective view of a system comprising at least two bone anchors with a receiving part and extension devices with different length coupled thereto.

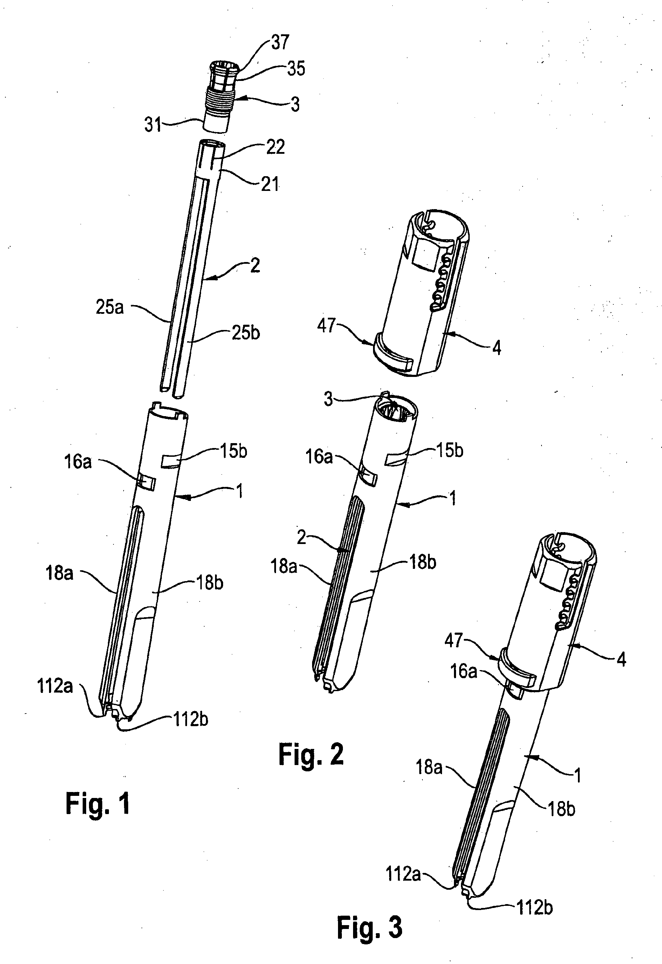

As shown in Figs. 1 to 3, an extension device according to a first embodiment includes a first sleeve 1 that forms an outer sleeve, a second sleeve 2 that forms an inner sleeve and that is positionable within the first sleeve 1 and an interlocking bushing 3. The interlocking bushing 3 is configured to be connected to the second sleeve 2 and is configured to couple the second sleeve 2 to the first sleeve 1 such as to permit a controlled motion of the second sleeve 2 relative to the first sleeve 1. In addition, a third sleeve 4 may be included in the extension device. The third sleeve 4 can be removably attached to the first sleeve 1 to extend the length of the first sleeve 1. When the first sleeve 1 and the second sleeve 2 are assembled as shown in Figs. 2 and 3, the extension device can be used to place the parts of a spinal stabilization system in a patient's body using a minimally invasive procedure. For some particular steps in the surgical procedure, the third sleeve 4 may be removed.

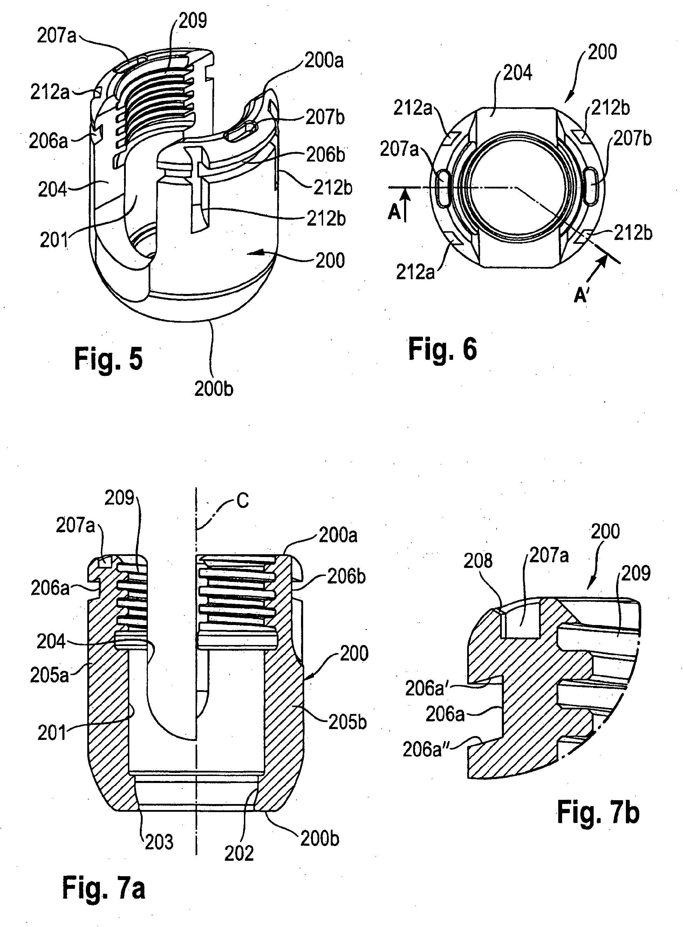

The extension device shown in Figs. 1 to 3 is configured to be used with a bone anchor for example with a pedicle screw. As depicted in Figs. 5 to 7b and 23a to 25b, an example of such a bone anchor may comprise an anchoring element with a threaded shank 100 and a spherical segment-shaped head 101, wherein the anchoring element is pivotably coupled to a receiving part 200. The receiving part is shown in more detail in Figs. 5 to 7b. Typically, the receiving part may be formed as a substantially cylindrical part with a first end or top end 200a and a second end or bottom end 200b, a central axis C, a coaxial bore 201 extending from the top end 200a to a distance from the bottom end 200b, and a seat 202 for the head 101 of the anchoring element as well as a lower opening 203 at the bottom end 200b where the shank of the bone anchoring element can pass through. A substantially U-shaped recess 204 may extend from the top end 200a in the direction of the bottom end 200b. The recess 204 serves for receiving a rod (not shown) therein. By means of the recess 204 two free legs 205a, 205b are formed. At an outer surface of the legs 205a, 205b at a distance from the top end 200a, circumferentially extending grooves 206a, 206b are formed that extend from one end of the channel formed by the U-shaped recess 204 to the other end and that are open towards the U-shaped recess 204. An upper sidewall 206a', 206b' and a lower sidewall 206a", 206b" of the groove 206a, 206b may be inclined towards the bottom end 200b.

The receiving part 200 may further include at the top end 200a in a free end surface of each of the legs 205a, 205b a recess 207a, 207b that extends into the legs in a direction parallel to the central axis C. The recess 207a, 207b is in a top view elongate and closed in a circumferential direction at both ends. Both recesses 207a, 207b serve for engagement with a portion of the extension device as described below. There may be a chamfered section 208 provided at the top end of each recess 207a, 207b that facilitates engagement of the recess 207a, 207b with a corresponding protrusion at the extension device.

In addition, the receiving part 200 may comprise a pair of longitudinal grooves 212a at one side of the U-shaped recess 204 and a pair of longitudinal grooves 212b at the opposite side. The grooves of each pair of grooves 212a, 212b are spaced apart from each other in the circumferential direction. Moreover, the grooves extend from the top end 200a in the outer wall of the receiving part on either side of the recesses 207a, 207b and are open towards the top end 200a. The grooves may have a decreasing depth towards their closed end that is located at a distance from the top end 200a. The cross-section of the longitudinal grooves 212a, 212b is substantially rectangular. As illustrated in particular in Fig. 5, the longitudinal grooves 212a, 212b intersect the circumferentially extending grooves 206a, 206b. The grooves 212a, 212b may interact with corresponding longitudinally extending pairs of ribs of the first sleeve in order to provide a form-fit connection.

In an upper portion of the legs 205a, 205b, an internal thread 209 may be provided for cooperating with a locking screw (not shown) for fixing the rod.

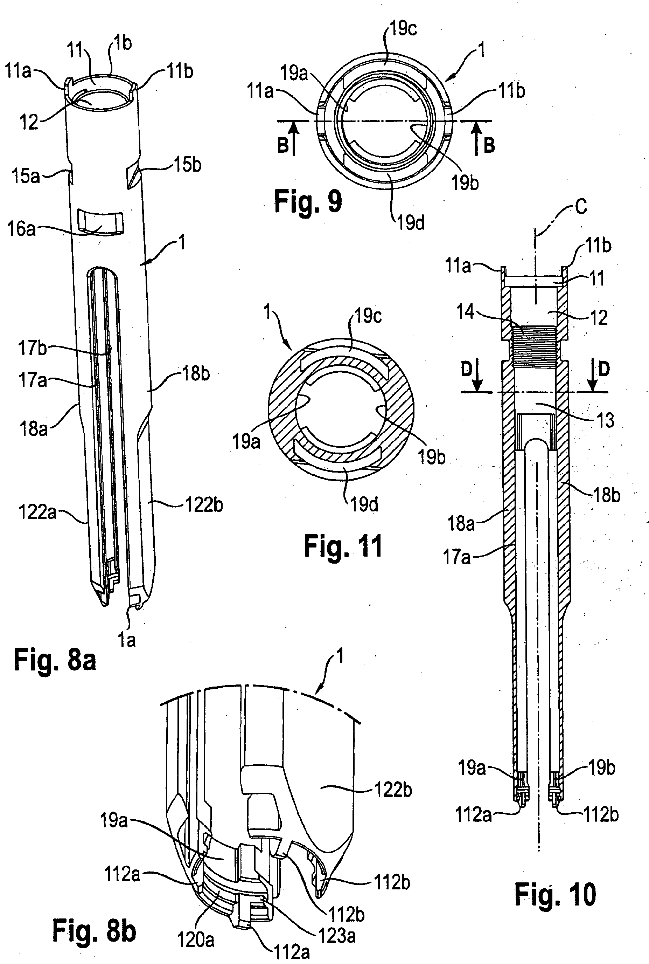

Referring to Figs. 8a to 10, the first sleeve 1 of the extension device is elongate and comprises a longitudinal axis c that is coaxial with the central axis C of the receiving part 200 when the extension device is coupled to the receiving part. The first sleeve 1 further has a front end 1a and a rear end 1b.

Adjacent to the rear end 1b, a first section 11 is provided with an inner diameter that is greater than an inner diameter of a following second section 12. Two small projections 11a, 11b protrude from the rear end 1b. They may interact with recesses in the third sleeve 4 for correct positioning of the third sleeve 4 onto the first sleeve 1. The second section 12 that follows the first section 11 serves the function of providing a clamping portion for the interlocking bushing 3 as described below. Adjacent to the second section 12, a third section 13 follows that may have a slightly smaller inner diameter compared to the second section 12. In the third section 13, an internal thread 15 is provided that is configured to interact with the interlocking bushing 3. The internally threaded section 15 is preferably adjacent to the second section 12. The first sleeve 1 may be in a portion thereof at 90° offset from the projections 11a, 11b double-walled by means of arc-shaped or cylinder segment-shaped longitudinal recesses 19c, 19d.

The first sleeve 1 may further comprise in its outer wall at a first distance from the rear end 1b two opposite first transverse recesses 15a, 15b that serve for attaching an instrument to the first sleeve 1. The recesses 15a, 15b may be at circumferential positions corresponding to the small projections 11a, 11b. Moreover, at a second distance from the rear end 1b two second recesses 16a, 16b may be provided, that extend in a circumferential direction and that are substantially 90° offset from the recesses 15a, 15b. The second transverse recesses 16a, 16b extend fully through an outermost wall of the first sleeve 1 and may serve for cleaning purposes to provide access to the recesses 19c, 19d.

Two elongate substantially U-shaped slits 17a, 17b that have a reverse U-shape compared to the slit 204 in the receiving part and that are offset from each other by 180° extend from the front end 1a towards the rear end 1b up to a distance from the second section 12 of the first sleeve 1. The longitudinal slits 17a, 17b have a width in a circumferential direction that is greater than a diameter of a spinal stabilization rod such that they permit to insert the rod through the slits. Preferably, the width of the slits 17a, 17b is substantially the same as the width of the U-shaped recess of the receiving part 200. The length of the slits 17a, 17b is typically more than 1/3 of the length of the first sleeve, preferably more than 1/2 of the length of the first sleeve. They can even be longer than 3/4 of the length of the first sleeve. The slits are located at a position that is offset by approximately 90° from the position of the first recesses 15a, 15b. By means of the slits 17a, 17b, two free legs 18a, 18b are formed that serve the purpose of interacting with the free legs 205a, 205b of the receiving part 200. By means of the legs 18a, 18b, the first sleeve 1 is slightly flexible in a direction perpendicular to the sleeve axis, so that the first sleeve 1 can be snapped onto the legs 205a, 205b of the receiving part 200.

The inner surface of the third section 13 of the first sleeve 1 comprises at each of the legs 18a, 18b a longitudinally extending substantially arc-shaped or cylinder segment-shaped guiding recess 19a, 19b into which a portion of the second sleeve 2 can extend to be guided therein. The guiding recesses 19a, 19b extend in a longitudinal direction beyond the slits 17a, 17b towards the rear end 1b, as can be seen in particular in Fig. 10.

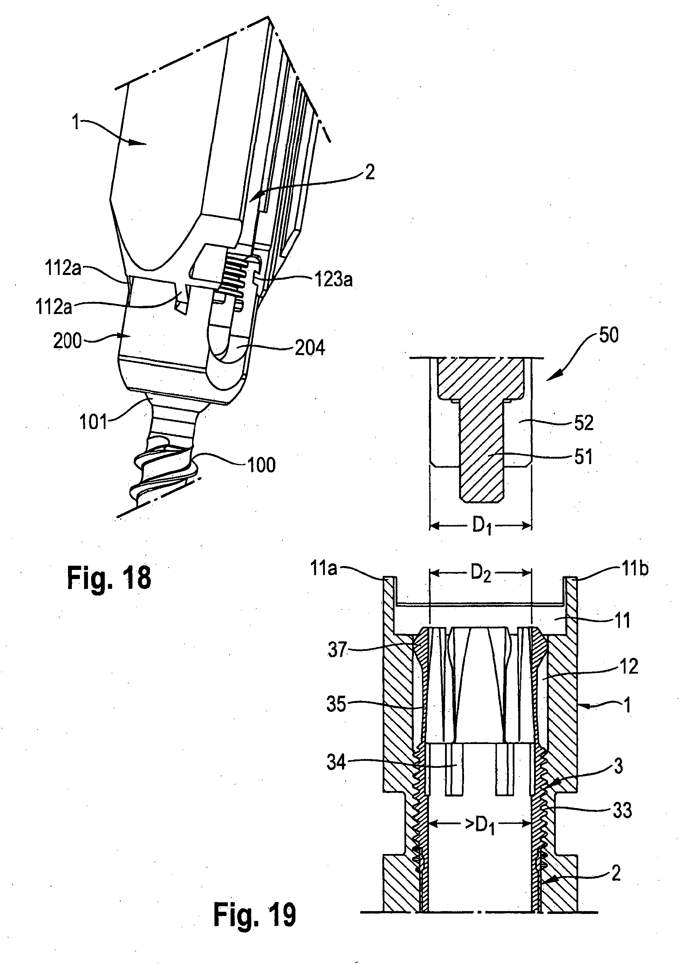

Each of the legs 18a, 18b comprises at a distance from the front end 1a an inwardly directed projection 120a, 120b that extends in a circumferential direction around the longitudinal axis c from one slit 17a to the opposite slit 17b. The shape of the circumferential projection 120a, 120b is substantially complementary to the shape of the circumferential grooves 206a, 206b of the receiving part 200, as can be seen in particular in Figs. 18 or 24a, 25a.

An upper surface of the projections 120a, 120b that faces towards the rear end 1b, may be inclined towards the rear end to match the complementary inclined upper wall 206a', 206b' of the groove 206a, 206b. A lower surface of the circumferential projections 120a, 120b may be perpendicular to the central axis or also inclined towards the rear end 1b. The inclination facilitates the engagement of the projections 120a, 120b into the groove 206a, 206b when the first sleeve 1 is coupled to the receiving part 200.

In addition, the first sleeve 1 comprises at positions that correspond in a circumferential direction to the positions of the longitudinal grooves 212a, 212b of the receiving part 200, a pair of ribs 112a, and a pair of ribs 112b on the legs 18a, 18b. The ribs 112a, 112b protrude inwards from the legs 18a, 18b and downwards from the front end 1a and have a shape that substantially matches the shape of the grooves 212a, 212b. As can be seen in particular in Fig. 8b, an outer surface of the ribs 112a, 112b tapers towards the free end, such that, when seen in a circumferential direction, the cross-section of the ribs is substantially triangular.

From each of the longitudinal ribs 112a, 112b, a transverse thickened rib portion 123a, 123b extends outward in a circumferential direction. This transverse rib portion 123a, 123b is located at a distance from the front end 1a that corresponds to the distance of the circumferentially extending groove 206a, 206b from the top end 200a. Hence, the transverse rib portion 123a, 123b is at the same level as the circumferential projections 120a, 120b.

By means of the longitudinal ribs 112a, 112b that are configured to engage the longitudinal grooves 212a, 212b, respectively, the strength of the connection between the head extension device and the receiving part is enhanced. The longitudinal ribs and grooves allow to apply a high torque with the extension device to the receiving part.

An overall outer shape of the first sleeve 1 may be cylindrical. Flattened outer surface portion 122a, 122b may be provided at an outer surface of the legs 18a, 18b for interacting with a tool (not shown).

The total length of the first sleeve is such that when the bone anchor is inserted into the bone and the first sleeve 1 is attached to the receiving part 200, the extension device protrudes to a sufficient extent from the operation site.

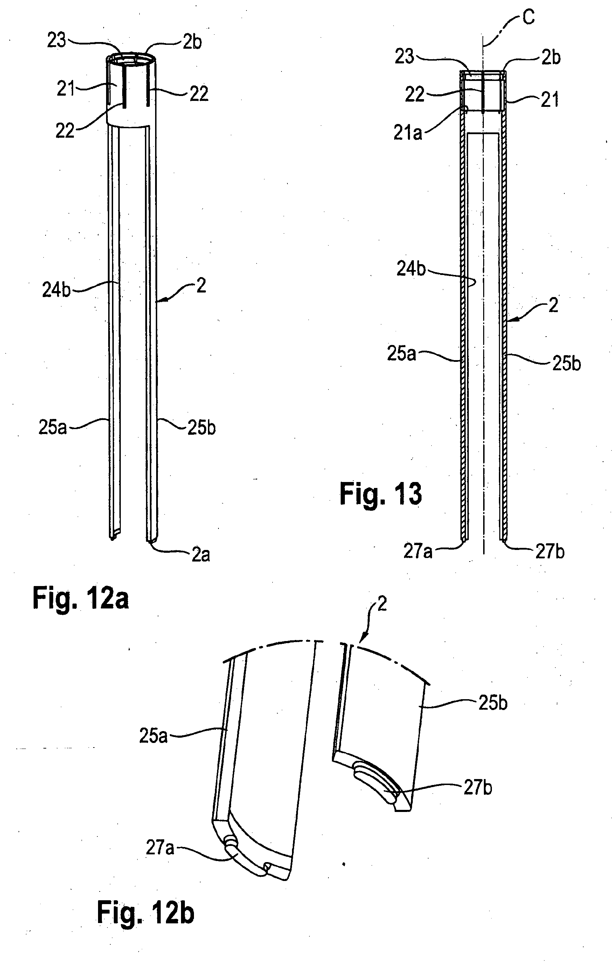

As illustrated in Figs. 12a to 13, the second sleeve 2 comprises a front end 2a and an opposite rear end 2b. The second sleeve 12 has a substantially constant outer diameter. Adjacent to the rear end 2b, the second sleeve 2 comprises a first portion 21 with a circumferentially closed cylinder surface. The first portion 21 is configured to engage the interlocking bushing 3. A plurality of longitudinal slits 22 that are open to the rear end 2b and extend up to a distance from the rear end 2b, are provided to render the first portion 21 flexible in such a way that the first portion 21 can elastically snap onto a portion of the interlocking bushing 3 and is held there by friction. At a first distance from the rear end 2b an inwardly extending annular projection 23, is provided. The annular projection 23 interacts with a corresponding depression or a groove at the interlocking bushing 3 to inhibit an inadvertent removal of the interlocking bushing 3 from the second sleeve 2. At a second distance from the rear end 2b that is greater than the first distance, a stop 21a, for example in the form of an annular shoulder, is provided. The shoulder 21a limits the insertion of the interlocking bushing into the second sleeve 2 and forms an abutment when the interlocking bushing is screwed downward into the first sleeve 1. An outer diameter of the first section 21 of the second sleeve 2 is smaller than an inner diameter of the third section 13 of the first sleeve 1.

Two recesses 24a, 24b with a substantially rectangular cross-section extend from the front end 2a through the second sleeve 2 up to the first portion 21. The recesses 24a, 24b have such a size that two opposite legs 25a, 25b are formed that fit into the guiding recesses 19a, 19b of the first sleeve 1. The legs 25a, 25b have such a length that they extend beyond the upper closed end of the recesses 17a, 17b of the first sleeve 1 in a direction towards the rear end 1b of the first sleeve 1.

The front end 2a of the second sleeve 2 is comprised of a substantially flat surface portion 26a, 26b on each of the legs 25a, 25b. This flat surface portion 26a, 26b is configured to cooperate with a substantially flat surface portion on the top end 200a of the receiving part. Moreover, a projections 27a, 27b on each leg that is configured to cooperate with the recess 207a, 207b at the free end of the legs of the receiving part 200 is provided. Hence, the projection 27a, 27b has a substantially complementary shape to the shape of the recess 207a, 207b. An inner surface of the projections 27a, 27b is flush with the inner surface of the legs 25a, 25b. An outer surface of the projections 27a, 27b is slightly recessed with respect to an outer surface of the legs 25a, 25b. The overall shape of the projections 27a, 27b is substantially arc-shaped with rounded edges corresponding to the recesses 207a, 207b in the receiving part. Moreover, in a circumferential direction, the projections 27a, 27b are arranged substantially in the middle of the legs 25a, 25b.

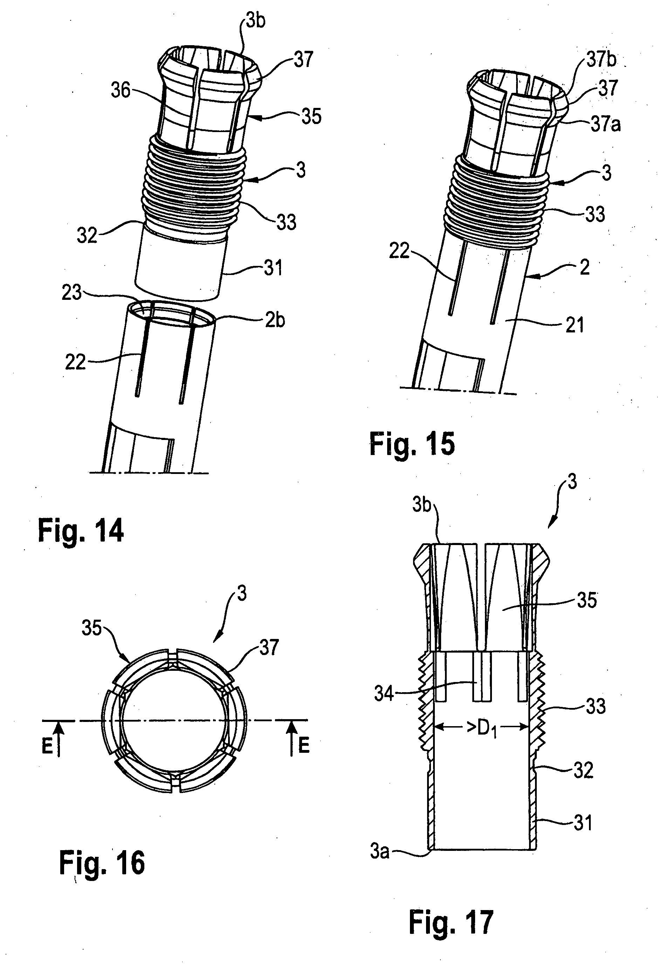

Turning now to Figs. 14 to 17, the interlocking bushing 3 comprises a front end 3a and a rear end 3b, a cylindrical section 31 adjacent to the front end 3a and that has an outer diameter substantially equal to an inner diameter of the first section 21 of the second sleeve 2 so that the bushing 3 is held in the second sleeve 2 by friction. Adjacent to the cylindrical section 31 with a smooth outer surface, there is a groove 32 that interacts with the annular projection 23 of the second sleeve 2. Following the groove 32 there is a threaded portion 33 with an external thread that is configured to interact with the threaded section 14 of the first sleeve 1.

The interlocking bushing 3 has an inner diameter in the threaded portion 33 that is slightly larger than D₁ wherein D₁ corresponds to an outer diameter of a decoupling instrument. A plurality of longitudinal engagement grooves 34 for a driver are provided in the inner wall of the interlocking bushing 3 at an upper region of the threaded portion 33. Adjacent to the rear end 3b, there is a collet portion 35 that is characterized by a plurality of substantially longitudinal slits 36 that are open towards the rear end 3b. The slits 36 render the collet portion 35 flexible, such that it can be radially compressed by exerting pressure from the outside. The collet portion 35 comprises at the rear end 3b of the interlocking bushing a thickened annular portion 37 that has a lower side 37a that tapers toward the sleeve axis and an upper side 37b that inversely tapers toward the sleeve axis so that a cross-section of the thickened portion 37 is substantially triangular. The length of the collet portion 35 is such that when the interlocking bushing 3 is screwed into the first sleeve 1 and the second sleeve 2 engages the receiving part 200, most part of the thickened portion 37 of the collet portion 35 is located within the second portion 12 of the first sleeve 1, as depicted more in detail in Fig. 19. In this position, the collet portion 35 is slightly compressed by the inner wall of the first sleeve 1 so that an inner diameter D₂ of the collet portion 35 is smaller than D₁ as shown in detail in Fig. 19. When the interlocking bushing 3 is screwed backward the collet portion 35 emerges from the second portion 12 whereby it expands.

Hence, the collet portion 35 forms a locking member that can assume a first configuration in which it is compressed and inhibits the insertion of a tool that removes the first sleeve 1 from the receiving part as long as the second sleeve 2 is still engaging the receiving part and that can assume a second configuration in which it is expanded and allows the insertion of the tool when the second sleeve 2 is decoupled. In this embodiment, the locking member is monolithically formed with the interlocking bushing 3.

The assembly of the second sleeve 2 and the interlocking bushing 3 is shown in Figs. 14 and 15. The interlocking bushing is pushed with its cylindrical portion 31 into the first section 21 of the second sleeve 2 until the annular projection 23 snaps into the groove 32. By means of this, the interlocking bushing 3 is coupled to the second sleeve 2 in such a manner, that a rotational motion of the interlocking bushing 3 relative to the second sleeve 2 is possible. An axial movement of the interlocking bushing 3 relative to the second sleeve 2 is inhibited when the interlocking bushing abuts against the stop 21 a provided in the second sleeve 2. The connection between the second sleeve 2 and the first sleeve 1 via the interlocking bushing 3 is a rigid connection.

The assembly comprising the second sleeve 2 and the interlocking bushing 3 in a mounted state as shown in Fig. 15 is then inserted into the first sleeve 1 from the rear end 1b until the threaded portion 33 of the interlocking bushing 3 engages the threaded section 14 of the first sleeve 1. The legs 25a, 25b are guided in the guiding recesses 19a, 19b in the first sleeve.

As depicted in Fig. 19, a release or decoupling instrument 50, only a front portion of which is shown, comprises an inner first portion 51 and an outer portion 52 that slidably receives the inner portion 51. The outer portion 52 may be expandable and serves for decoupling the first sleeve 1 from the receiving part 200. It has an outer diameter D₁ so that the outer portion 52 can be guided through the interlocking bushing 3 into the first sleeve 1 and can be spread there. Thereby, the first sleeve 1 can be decoupled from the receiving part.

The decoupling instrument 50 can be inserted into the interlocking bushing 3 only, if the collet portion 35 is in the second configuration, where it protrudes outward of the second portion 12 into the first portion 11 of the first sleeve 1. Because the first portion 11 has a greater inner diameter than the second portion 12, the collet portion 35 can expand therein so that the inner diameter is sufficiently large to allow insertion of the decoupling instrument 50.

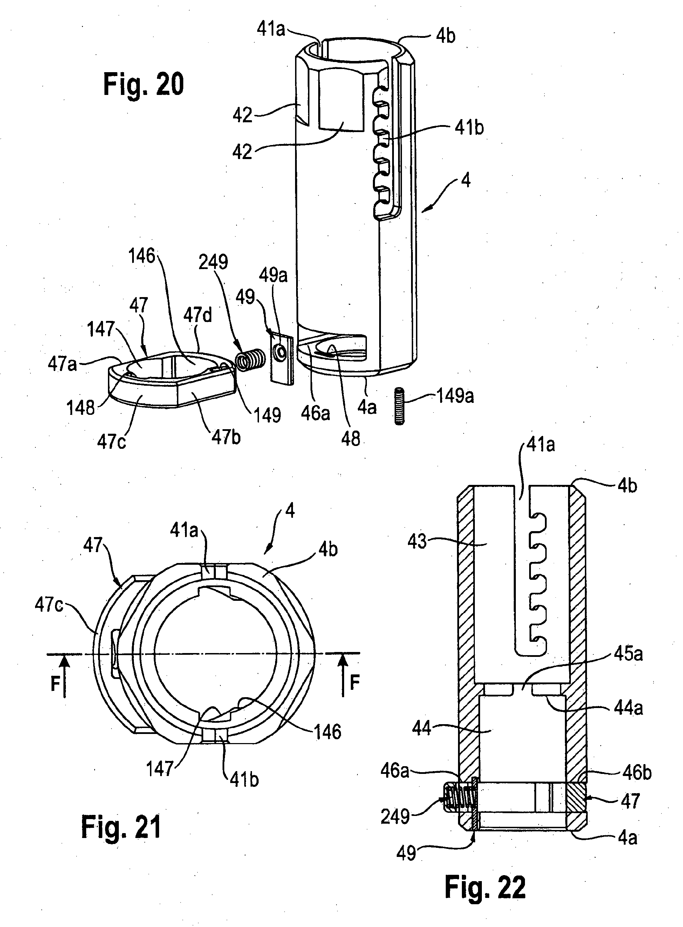

Referring to Figs. 20 to 22, the optional third sleeve 4 will be described. The third sleeve 4 has a first or front end 4a and a second or rear end 4b. Its outer diameter is preferably greater.than the outer diameter of the first sleeve 1. Adjacent to its rear end 4b it comprises two opposite longitudinal slits 41a, 41b that extend from the second end 4b along at least a portion of the third sleeve that may be up to 1/2 of the length of the third sleeve. One of the sidewalls of each longitudinal slit 41a, 41b comprises a wavy structure for latching with a reduction sleeve (not shown) used for further steps in the surgical procedure, for example for pressing down the rod and inserting a locking screw to fix the rod. Furthermore, an engagement structure 42, for example, a plurality of flat engagement portions, is provided at an outer surface of an upper portion of the third sleeve for applying a tool thereto.

The third sleeve 4 has a first inner portion 43 adjacent to the rear end 4b and a second inner portion 44 adjacent to the lower end 4a. The second inner portion 44 is configured to accommodate the upper portion of the first sleeve 1 therein. For this purpose, the inner diameter of the second section 44 is slightly larger than the outer diameter of the upper end of the first sleeve 1. An upper end of the second portion 44 may provide a shoulder 44a, 44b that forms an abutment for the rear end 1b of the first sleeve. The shoulder may have an interruption at positions 45a, 45b that correspond to the small protrusions 11a, 11b of the first sleeve. An inner diameter of the first portion 43 may be larger than an inner diameter of the second portion 44.

At a distance from the lower end 4a two transverse slots 46a, 46b are provided at positions that are 180° offset from each other. The slots 46a, 46b are configured to accommodate an operating pusher 47 therein. The slots 46a, 46b are elongate in a circumferential direction and preferably positioned at 90° with respect to the longitudinal slits 41a, 41b. Moreover, in the second section 44, there is at the bottom of one of the elongate transverse slots 46a a substantially rectangular recess 48 that serves for accommodating an abutment plate 49 therein.

The pusher 47 is a flat piece that can extend through the slots 46a, 46b. It has two substantially parallel longitudinal outer walls 47a, 47b and two outwardly curved sidewalls 47c, 47d that connect the parallel longitudinal sidewalls 47a, 47b, respectively. A total length from the outer end of one curved sidewall 47c to the opposite curved sidewall 47b is greater than the outer diameter of the third sleeve 4 at the position of the transverse slots 46a, 46b, so that a portion of the pusher 47 protrudes outward when the pusher is inserted into the slots 46a, 46b of the third sleeve 4. The inside of the pusher is hollow to accommodate the upper portion of the first sleeve 1 therein. More in detail, the pusher 47 has a first inner portion 146 with an inner contour that matches an outer contour of the upper portion of the first sleeve 1 and adjacent thereto a second inner portion 147 with an inner contour greater than an outer contour of the first sleeve 1.

At an inner side of the curved outer sidewall 47c a recess 148 for accommodating a spring 249 is provided. Furthermore, on one of the substantially flat long sides 47a, 47b, preferably near the other curved sidewall 47d, an elongate hole 149 is provided that is elongate in the lengthwise direction in which the pusher 47 can be moved. A securing element 149a, such as a headless screw, extends through the wall of the third sleeve 4 into the elongate recess 149 and limits the path of movement of the pusher 47 relative to the third sleeve 4 and prevents loss of the pusher 47.

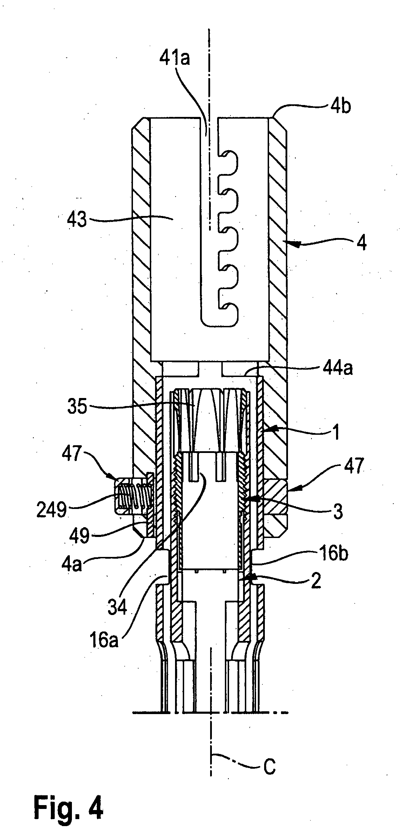

The spring 249, that may be a helical spring as shown or any other kind of spring, is positioned in the recess 148 of the pusher 47 and extends into a counter-sink 49a in the abutment plate 49. The spring 249 is biased such, that it holds the pusher 47 in a position in which one curved sidewall 47c protrudes outward from the transverse slot 46a and the opposite curved sidewall 47d is within the opposite slot 46b. In this position, the portion 147 that has the larger inner diameter of the pusher 47 is partially narrowed by the third sleeve 4, as can be seen in Fig. 21. Hence, in this position, the first sleeve 1 fits into the portion 44 of the third sleeve 4 and is held via the pusher 47 by means of friction, as can be also seen in Fig. 4. To remove the third sleeve 4 from the first sleeve 1, the pusher 47 that protrudes outward from the slot 46a, is pushed against the spring force into the slot 46a so that the opposite curved outer wall 47d protrudes from the opposite slot 46b. By means of this, the inner section 147 that has a larger diameter than the upper portion of the first sleeve 1 is brought into a position around the first sleeve 1. In this position, the third sleeve 4 can be removed by pulling the third sleeve 4 from the first sleeve 1.

The mounting of the third sleeve 4 onto the first sleeve 1 is carried out in a similar manner by pushing the pusher 47 into the slot 46a and placing the third sleeve 4 out the first sleeve.

The parts of the extension device are all made of a body-compatible material, such as titanium or stainless steel, a body-compatible metal alloy, for example Ti-Ni-alloy, such as Nitinol, or a body-compatible plastic material, such as PEEK. The parts may be all of the same or of different materials.

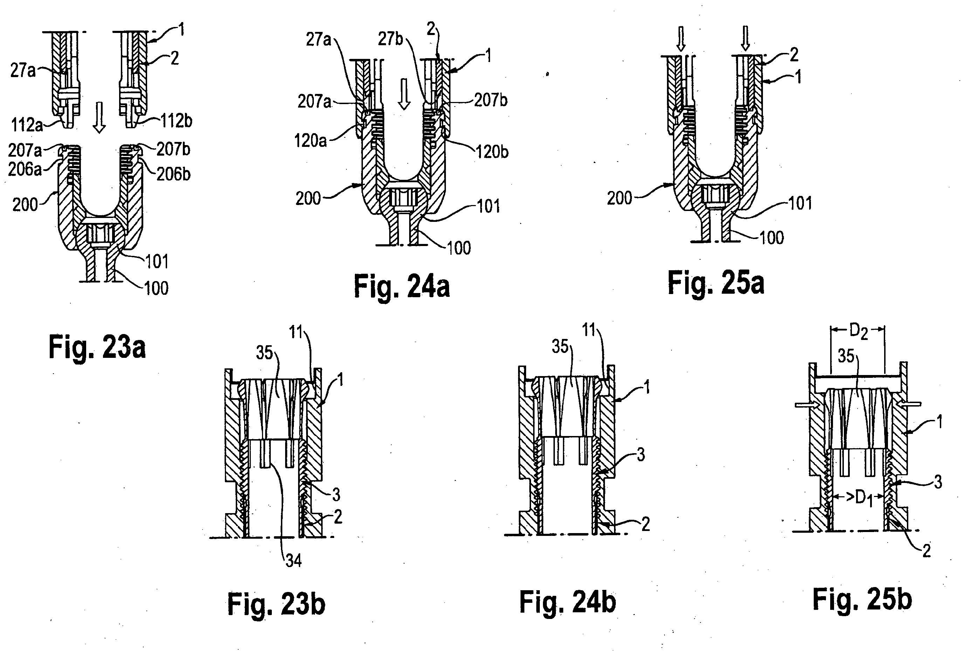

Referring now to Figs. 18 to 25b, the attachment of the extension device to a bone anchor will be described. As shown in Fig. 23a, the extension device is moved with the front end 1a of the first sleeve 1 toward the receiving part 200. Then, the legs 18a, 18b of the first sleeve 1 are spread to a certain extent when they touch the top end 200a of the receiving part 200 and by further downward movement of the extension device, the circumferential projections 120a, 120b of the legs 18a, 18b snap into the circumferential grooves 206a, 206b. Also, the vertical ribs 112a, 112b engage the corresponding vertical grooves 212a, 212b of the receiving part 200. During coupling of the first sleeve 1, the second sleeve 2 is in a retracted position, in which the collet portion 35 extends into the first portion 11 of the first sleeve 1. This configuration is shown in Figs. 23a to 24b. The thickened upper edge 37 of the collet portion 35 protrudes out of the second portion 12 of the first sleeve so that an inner diameter of the collet portion 35 is at least a diameter >D₁ corresponding to an inner diameter of the threaded portion 33 of the interlocking bushing 3.

Then, as shown in Figs. 25a and 25b, the second sleeve 2 is moved relative to the first sleeve 1 towards the receiving part 200 by screwing the interlocking bushing 3 further towards the front end 1a of the first sleeve 1. When the projection 27a, 27b of the second sleeve 2 enter the corresponding recesses 207a, 207b at the top end 200a of the receiving part 200, a rotational movement of the second sleeve 2 relative to the first sleeve 1 and therefore also relative to the receiving part 200 is inhibited. At the same time, the tapered upper thickened portion 37 of the collet portion 35 slides along the shoulder formed by the transition between the first portion 11 and the second portion 12 of the first sleeve 1 until it enters the second portion 12. Thereby, the collet portion 35 is radially compressed as shown in Fig. 25b. An upper inner diameter of the collet portion is now D₂. The second sleeve 2 can rotate relative to the bushing 3, so that the alignment between the legs 18a, 18b of the first sleeve 1 and the legs 25a, 25b of the second sleeve 2 is maintained. A further rotation of the interlocking bushing 3 presses the flat surface portions 26a, 26b of the front end 2a of the second sleeve 2 onto the free flat end surfaces of the receiving part 200. Thereby and by the engagement of the projections 120a, 120b, 112a, 112b of the first sleeve 1 in the grooves 206a, 206b, 212a, 212b of the second sleeve 2 the first sleeve 1 is interlocked with the receiving part 200 and with the second sleeve 2 to provide a safe and strong connection between the extension device and the receiving part. In such a configuration, the insertion of the rod and the locking screw can take place as well as surgical steps thereafter such as compression and distraction steps using the extension device.

The decoupling instrument that comprises an outer diameter D₁ cannot be inserted in the configuration shown in Fig. 25b. Hence, the collet portion 35 acts as a locking member that prevents the decoupling of the extension device when the second sleeve 2 is coupled to the receiving part. This enhances the safety of the procedure and prevents damage of portions of the extension device, in particular of the inner sleeve 2.

Turning the interlocking bushing 3 in the opposite direction, releases the interlocking connection and permits to retract the projections 27a, 27b of the second sleeve out of the recesses 207a,207b.

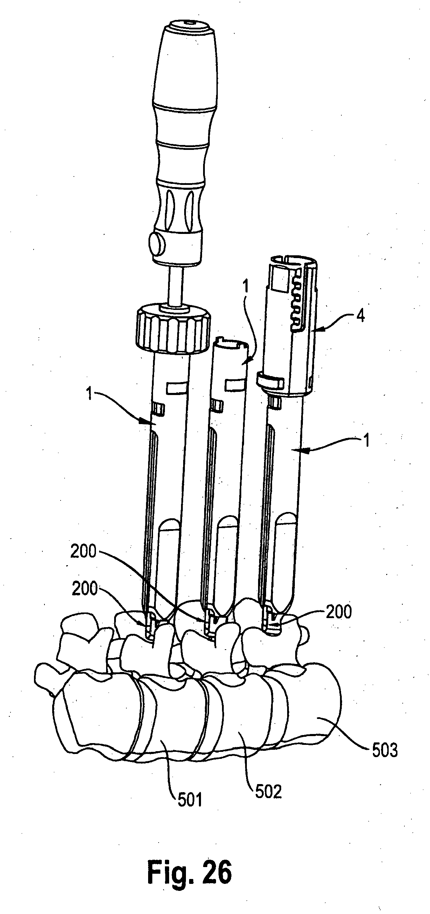

In the clinical use, as shown in Fig. 26, the extension device is attached to a receiving part 200 of a bone anchor. The whole bone anchor is inserted through a minimally invasive procedure into a pedicle of a vertebra. Exemplary, three vertebrae 501, 502, 503 are shown with bone anchors and mounted extension devices. By rotating the extension devices with the aid of an instrument, the channels 204 of the receiving parts of the bone anchors can be aligned to permit the insertion of a rod. Because the connection between the receiving part 200 and the extension device is robust and safe, an easy alignment using the extension devices is possible. Thereafter, the rod is inserted through the slits 17a, 17b of the extension devices (not shown) and fixed with a locking screw that is guided through the extension device until it can be screwed between the legs 205a, 205b of the receiving part. By applying an instrument to the extension device, a compression or distraction procedure can be performed using a minimally invasive technique. Using only the first sleeve 1 and the second sleeve 2 without the third sleeve 4, has the advantage that the extension devices have a greater distance with respect to each other and that a greater angle between one extension device and another extension device can be achieved. The third sleeve 4 may be used in cases in which the surgeon has to approximate the rod.

Modifications of the above described embodiment may be contemplated. It shall be noted, that the shape of the engaging complementary structures of the first sleeve and the receiving part as well as the second sleeve and the receiving part can be modified and are not limited to the exact shape shown in the embodiments. In a further modification, the second sleeve may be coupled to the receiving part by only a frictional engagement of a portion of the second sleeve and a portion of the receiving part. For example, a front end surface of the second sleeve may be pressed against a free end surface of the legs 205a, 205b of the receiving part. In a further modification the first sleeve may be coupled to the receiving part by such a friction-fit.

The function of the first and the second sleeve may be interchanged. In such a case, the locking member inhibits the decoupling of the second sleeve when the first sleeve is still coupled to the receiving part.

The extension device can be used with any bone anchor that comprises a receiving part, such as a polyaxial bone anchor, a monoaxial bone anchor and can also be used with different shapes of receiving parts. Anchors with inner compression members or outer rings may be used. The only necessity is an engagement structure at the receiving part that can cooperate with a corresponding engagement structure of the extension device.

The locking member in the form of a collet portion is shown as a monolithic part of the interlocking bushing. However, a locking member can also be a separate member, for example a separate collet. Other kinds of locking members that can be actuated by actuating the second sleeve, may also be conceivable.

## Claims

1. A system of an extension device for a bone anchor and a bone anchor,
wherein the bone anchor comprises
an anchoring section (100) for anchoring in a bone and
a receiving part (200) connected to the anchoring section, the receiving part comprising a central axis (C) and a channel (204) for receiving a rod, wherein sidewalls of the channel form two free legs (205a, 205b),
the extension device comprising
a first sleeve (1) with a first sleeve axis (c) that is coaxial to the central axis (C), wherein the first sleeve (1) is configured to be coupled to the receiving part (200) and decoupled from the receiving part (200);
a second sleeve (2) with a second sleeve axis (c) coaxial to the central axis (C), wherein the second sleeve (2) is positioned within the first sleeve (1) and positionable relative to the first sleeve along the central axis (C) and wherein the second sleeve (2) is configured to be coupled to the receiving part (200) and decoupled from the receiving part (200);
a locking member that in a first configuration inhibits decoupling of one of the first sleeve (1) or the second sleeve (2) from the receiving part (200) when the other one of the first sleeve (1) or the second sleeve (2) is still coupled to the receiving part,
wherein the locking member is formed as a collet or collet portion (35) that is positioned at least partially inside the second sleeve (2) and comprises a spring that is compressible and/or extendible in a radial direction relative to the central axis (C), wherein the collet portion 35 can assume the first configuration in which it is compressed and inhibits insertion of a decoupling instrument (50), and can assume a second configuration in which it is expanded and allows the insertion of the decoupling instrument (50).

2. The system of claim 1, wherein in the second configuration the locking member permits decoupling of one of the first sleeve (1) or the second sleeve (2) from the receiving part (200) when the other one of the first sleeve (1) or the second sleeve (2) is already decoupled from the receiving part.

3. The system of claim 1 or 2, wherein the locking member (35) inhibits decoupling of the first sleeve (1) when the second sleeve (2) is coupled to the receiving part.

4. The system of one of claims 1 to 3, wherein the locking member is sized and arranged so as to inhibit attachment of a decoupling instrument when the locking member is in the first configuration.

5. The system of one of claims 1 to 4, wherein the locking member comprises a spring that is compressible and/or extendible in a radial direction relative to the central axis (C).

6. The system of one of claims 1 to 5, wherein the second sleeve (2) is connected to the first sleeve (1) through a coupling member (3) that is configured to advance together with the second sleeve (2) in an axial direction relative to the first sleeve (1).

7. The system of claim 6, wherein the coupling member (3) is a bushing that is coupled to the first sleeve (1) through an advancement structure (33, 14) that permits to advance the coupling member with the second sleeve (2) to a given position.

8. The system of one of claims 6 to 7, wherein the coupling member (3) is coupled to the second sleeve (2) so that it can rotate with respect to the second sleeve.

9. The system of one of claims 6 to 8, wherein the locking member and the coupling member (3) are formed as a single monolithic part.

10. The system of one of claims 1 to 9, further comprising a third sleeve (4) that is selectively connectable to the first sleeve (1) to enlarge an axial length of the extension device.

11. The system of claim 10, further comprising a bushing (3) configured to be connected to the second sleeve (2) and configured to couple the second sleeve (2) relative to the first sleeve (1) such as to permit a controlled motion of the second sleeve (2) relative to the first sleeve (1).

12. The system one of claims 1 to 11, wherein the the receiving part (200) further comprises an engagement structure (206a, 206b, 212a, 212b) that can be coupled to a substantially complementary engagement structure (120a, 120b, 112a, 112b) of the extension device.

13. The system of claim 12, wherein the both, the first sleeve (1) and the second sleeve (2) comprise an engagement structure (120a, 120b, 112a, 112b; 27a, 27b) for a form-fit engagement with a corresponding engagement structure (206a, 206b, 212a, 212b; 207a, 207b) of the receiving part (200).

14. The system of claim 12, wherein the first sleeve (1) comprises an engagement structure (120a, 120b, 112a, 112b) for a form-fit engagement with a corresponding engagement structure (206a, 206b, 212a, 212b) of the receiving part and wherein the second sleeve (2) is configured to be coupled to the receiving part(200) via a frictional engagement.

15. A spinal stabilization system for use with minimally invasive surgery comprising a system of one of claims 1 to 14 with at least two bone anchors and an extension device for each bone anchor.

## Patentansprüche

1. Ein System aus einer Verlängerungsvorrichtung für einen Knochenanker und einen Knochenanker,
wobei der Knochenanker umfasst:
einen Verankerungsabschnitt (100) zum Verankern in einem Knochen, und
ein Aufnahmeteil (200), das mit dem Verankerungsabschnitt verbunden ist, wobei das Aufnahmeteil eine Mittenachse (C) und einen Kanal (204) zum Aufnehmen eines Stabs umfasst, wobei Seitenwände des Kanals zwei freie Schenkel (205a, 205b) ausbilden,
wobei die Verlängerungsvorrichtung umfasst:
eine erste Hülse (1) mit einer ersten Hülsenachse (c), die koaxial zur Mittenachse (C) ist, wobei die erste Hülse (1) eingerichtet ist, mit dem Aufnahmeteil (200) verkoppelt und von dem Aufnahmeteil (200) entkoppelt zu werden;
eine zweite Hülse (2) mit einer zweiten Hülsenachse (c), die koaxial zu der Mittenachse (C) ist, wobei die zweite Hülse innerhalb der ersten Hülse (1) positioniert ist und relativ zu der ersten Hülse entlang der Mittenachse (C) positioniert werden kann, und wobei die zweite Hülse (2) eingerichtet ist, mit dem Aufnahmeteil (200) verkoppelt und von dem Aufnahmeteil (200) entkoppelt zu werden;
ein Verriegelungselement, das in einer ersten Konfiguration eine Entkopplung der ersten Hülse (1) oder der zweiten Hülse (2) von dem Aufnahmeteil (200) verhindert, wenn die jeweils andere erste Hülse (1) oder zweite Hülse (2) noch mit dem Aufnahmeteil verkoppelt ist,
wobei das Verriegelungselement als eine Klemmhülse oder ein Klemmhülsenabschnitt (35) ausgebildet ist, die/der zumindest teilweise innerhalb der zweiten Hülse (2) positioniert ist und eine Feder umfasst, die in einer radialen Richtung relativ zur Mittenachse (C) komprimier- und/oder dehnbar ist, wobei der Klemmhülsenabschnitt (35) die erste Konfiguration einnehmen kann, in welcher sie komprimiert ist und das Einführen eines Entkopplungsinstruments (50) verhindert, und eine zweite Konfiguration einnehmen kann, in welcher sie expandiert ist und das Einführen des Entkopplungsinstruments (50) ermöglicht.

2. Das System gemäß Anspruch 1, wobei das Verriegelungselement eine Entkopplung der ersten Hülse (1) oder der zweiten Hülse (2) von dem Aufnahmeteil (200) in der zweiten Konfiguration ermöglicht, wenn die jeweils andere erste Hülse (1) oder zweite Hülse (2) bereits von dem Aufnahmeteil entkoppelt ist.

3. Das System gemäß Anspruch 1 oder 2, wobei das Verriegelungselement (35) eine Entkopplung der ersten Hülse (1) verhindert, wenn die zweite Hülse (2) mit dem Aufnahmeteil verkoppelt ist.

4. Das System gemäß einem der Ansprüche 1 bis 3, wobei das Verriegelungselement eine Größe besitzt und eingerichtet ist, so dass ein Anbringen eines Entkopplungsinstruments verhindert wird, wenn sich das Verriegelungselement in der ersten Konfiguration befindet.

5. Das System gemäß einem der Ansprüche 1 bis 4, wobei das Verriegelungselement eine Feder umfasst, die komprimierbar und/oder dehnbar in einer radialen Richtung relativ zu der Mittenachse (C) ist.

6. Das System gemäß einem der Ansprüche 1 bis 5, wobei die zweite Hülse (2) mit der ersten Hülse (1) durch ein Kopplungselement (3) verbunden ist, das eingerichtet ist, zusammen mit der zweiten Hülse (2) in einer axialen Richtung relativ zu der ersten Hülse (1) vorbewegt zu werden.

7. Das System gemäß Anspruch 6, wobei das Kopplungselement (3) eine Buchse ist, die mit der ersten Hülse (1) durch eine Vortriebsstruktur (33, 14) verkoppelt ist, die es erlaubt, das Kopplungselement mit der zweiten Hülse (2) zu einer vorgegebenen Position vorzutreiben.

8. Das System gemäß einem der Ansprüche 6 bis 7, wobei das Kopplungselement (3) mit der zweiten Hülse (2) verkoppelt ist, so dass es in Bezug auf die zweite Hülse rotieren kann.

9. Das System gemäß einem der Ansprüche 6 bis 8, wobei das Verriegelungselement und das Kopplungselement (3) als ein einzelnes monolithisches Teil ausgebildet sind.

10. Das System gemäß einem der Ansprüche 1 bis 9, ferner umfassend eine dritte Hülse (4), die wahlweise mit der ersten Hülse (1) verbindbar ist, um eine axiale Länge der Verlängerungsvorrichtung zu vergrößern.

11. Das System gemäß Anspruch 10, ferner umfassend eine Buchse (3), die eingerichtet ist, mit der zweiten Hülse (2) verbunden zu werden, und die eingerichtet ist, die zweite Hülse (2) relativ zu der ersten Hülse (1) zu verkoppeln, so dass eine kontrollierte Bewegung der zweiten Hülse (2) relativ zu der ersten Hülse (1) ermöglicht wird.

12. Das System gemäß einem der Ansprüche 1 bis 11, wobei das Aufnahmeteil (200) ferner eine Eingriffsstruktur (206a, 206b, 212a, 212b) umfasst, die an eine im Wesentlichen komplementäre Eingriffsstruktur (120a, 120b, 112a, 112b) der Verlängerungsvorrichtung angekoppelt werden kann.

13. Das System gemäß Anspruch 12, worin beide, die erste Hülse (1) und die zweite Hülse (2) eine Eingriffsstruktur (120a, 120b, 112a, 112b; 27a, 27b) für einen formschlüssigen Eingriff durch eine entsprechende Eingriffsstruktur (206a, 206b, 212a, 212b; 207a, 207b) des Aufnahmeteils (200) umfassen.

14. Das System gemäß Anspruch 12, wobei die erste Hülse (1) eine Eingriffsstruktur (120a, 120b, 112a, 112b) für einen formschlüssigen Eingriff durch eine entsprechende Eingriffsstruktur (206a, 206b, 212a, 212b) des Aufnahmeteils umfasst, und wobei die zweite Hülse (2) eingerichtet ist, an das Aufnahmeteil (200) über einen reibschlüssigen Eingriff angekoppelt zu werden.

15. Ein Wirbelsäulenstabilisierungssystem zur Verwendung in minimalinvasiver Chirurgie umfassend ein System gemäß einem der Ansprüche 1 bis 14 mit wenigstens zwei Knochenankern und einer Verlängerungsvorrichtung für jeden Knochenanker.

## Revendications

1. Système de dispositif d'extension pour un ancrage osseux et un ancrage osseux, l'ancrage osseux comprenant :
une section d'ancrage (100) destinée à être ancrée dans un os, et
une partie de réception (200) raccordée à la section d'ancrage, la partie de réception comprenant un axe central (C) et un canal (204) pour recevoir une barre, les parois latérales du canal formant deux branches libres (205a, 205b),
le dispositif d'extension comprenant
un premier manchon (1) avec un premier axe de manchon (c) coaxial par rapport à l'axe central (C), le premier manchon (1) étant configuré pour être accouplé à la partie de réception (200) et désaccouplé de la partie de réception (200) ;
un deuxième manchon (2) avec un deuxième axe de manchon (c) coaxial par rapport à l'axe central (C), le deuxième manchon (2) étant positionné à l'intérieur du premier manchon (1) et pouvant être positionné par rapport au premier manchon le long de l'axe central (C), le deuxième manchon (2) étant configuré pour être accouplé à la partie de réception (200) et désaccouplé de la partie de réception (200);
un élément de verrouillage qui, dans une première configuration, empêche le désaccouplement de l'un des manchons, soit du premier manchon (1) ou du deuxième manchon (2), de la partie de réception (200) lorsque l'autre manchon, soit le deuxième manchon (2) ou le premier manchon (1), est toujours accouplé à la partie de réception,
l'élément de verrouillage étant configuré en collet ou en portion de collet (35) positionné au moins en partie à l'intérieur du deuxième manchon (2) et comprenant un ressort qui peut être comprimé et/ou détendu dans une direction radiale par rapport à l'axe central (C), la portion de collet 35 pouvant adopter la première configuration dans laquelle elle est comprimée et empêche l'insertion d'un instrument de désaccouplement (50), et pouvant adopter une deuxième configuration dans laquelle elle est dilatée et permet l'insertion de l'instrument de désaccouplement (50).

2. Système selon la revendication 1, dans lequel, dans la deuxième configuration, l'élément de verrouillage permet le désaccouplement de l'un des manchons, soit du premier manchon (1) ou du deuxième manchon (2), de la partie de réception (200) lorsque l'autre manchon, soit le deuxième manchon (2) ou le premier manchon (1), est déjà désaccouplé de la partie de réception.

3. Système selon la revendication 1 ou 2, dans lequel l'élément de verrouillage (35) empêche le désaccouplement du premier manchon (1) lorsque le deuxième manchon (2) est accouplé à la partie de réception.

4. Système selon l'une des revendications 1 à 3, dans lequel l'élément de verrouillage est dimensionné et agencé de manière à empêcher la fixation d'un instrument de désaccouplement lorsque l'élément de verrouillage est dans la première configuration.

5. Système selon l'une des revendications 1 à 4, dans lequel l'élément de verrouillage comprend un ressort qui peut être comprimé et/ou détendu dans une direction radiale par rapport à l'axe central (C).

6. Système selon l'une des revendications 1 à 5, dans lequel le deuxième manchon (2) est relié au premier manchon (1) par l'intermédiaire d'un élément d'accouplement (3) qui est configuré pour avancer en même temps que le deuxième manchon (2) dans une direction axiale par rapport au premier manchon (1).

7. Système selon la revendication 6, dans lequel l'élément de d'accouplement (3) est une bague accouplée au premier manchon (1) par l'intermédiaire d'une structure d'avancement (33, 14) qui permet d'avancer l'élément d'accouplement avec le deuxième manchon (2) jusqu'à une position donnée.

8. Système selon l'une des revendications 6 à 7, dans lequel l'élément d'accouplement (3) est accouplé au deuxième manchon (2) de manière à pouvoir pivoter par rapport au deuxième manchon.

9. Système selon l'une des revendications 6 à 8, dans lequel l'élément de verrouillage et l'élément d'accouplement (3) sont formés en une seule pièce monolithique.

10. Système selon l'une des revendications 1 à 9, comprenant en outre un troisième manchon (4) qui peut être relié sélectivement au premier manchon (1) pour agrandir une longueur axiale du dispositif d'extension.

11. Système selon la revendication 10, comprenant en outre une bague (3) configurée pour être reliée au deuxième manchon (2) et configurée pour accoupler le deuxième manchon (2) par rapport au premier manchon (1) de manière à permettre un déplacement contrôlé du deuxième manchon (2) par rapport au premier manchon (1).

12. Système selon l'une des revendications 1 à 11, dans lequel la partie de réception (200) comprend en outre une structure d'engagement (206a, 206b, 212a, 212b) qui peut être accouplée à une structure d'engagement (120a, 120b, 112a, 112b) substantiellement complémentaire du dispositif d'extension.

13. Système selon la revendication 12, dans lequel le premier manchon (1) et le deuxième manchon (2) comprennent tous deux une structure d'engagement (120a, 120b, 112a, 112b; 27a, 27b) pour un engagement par complémentarité de forme avec une structure correspondante d'engagement (206a, 206b, 212a, 212b; 207a, 207b) de la partie de réception (200).

14. Système selon la revendication 12, dans lequel le premier manchon (1) comprend une structure d'engagement (120a, 120b, 112a, 112b) pour un engagement par complémentarité de forme avec une structure correspondante d'engagement (206a, 206b, 212a, 212b) de la partie de réception, et dans lequel le deuxième manchon (2) est configuré pour être accouplé à la partie de réception (200) par un engagement par frottement.

15. Système de stabilisation de la colonne vertébrale destiné à être utilisé en chirurgie minimalement invasive, comprenant un système selon l'une des revendications 1 à 14 présentant au moins deux ancrages osseux et un dispositif d'extension pour chaque ancrage osseux.
